# EUROPEAN PATENT APPLICATION

(11) **EP 4 324 485 A1**
(43) Date of publication of application: **21.02.2024**
(21) Application number: 22190407.1
(22) Date of filing: 15.08.2022
(51) Int. Cl.: A61K 48/00, C08G 73/02

(54) **A METHOD FOR THE PRODUCTION OF PEGYLATED HYPERBRANCHED POLY( -AMINO ESTER)S, PEGYLATED HYPERBRANCHED POLY( -AMINO ESTER)S PRODUCED BY THOSE METHODS AND THEIR USE IN ENHANCED NUCLEIC ACID DELIVERY**

(71) Applicant: Branca Banus Ltd, Dublin D04V1W8 (IE)
(72) Inventor: Wang, Wenxin, Dublin (IE); He, Zhonglei, Dublin (IE); Li, Yinghao, Dublin (IE); A, Sigen, Dublin (IE); Tai, Hongyun, Dublin (IE)
(74) Representative: Tomkins & Co

(57) **Abstract**

The present invention relates to PEG-modified hyperbranched poly(β-amino ester)s and a method for their synthesis. It further relates to polyplexes made with the PEG-modified HPAEs and a method for producing same. These polyplexes are useful in *in vivo or in vitro* transfection processes.

## Description

### Field of the Invention

The present invention relates to a method for PEGylation of hyperbranched poly(β-amino ester)s. It further relates to polyplexes made with the resulting PEG-modified HPAEs and a method for producing same. These polyplexes may be used in transfection processes, in particular *in vivo* transfection processes.

### Background to the Invention

Numerous diseases arise due to abnormalities in an individual's genetic code, leading to mutations that culminate in genetic disorders. Such genetic diseases account for considerable social and economic burdens globally and lead to severe lifelong negative impacts on the lives of suffering patients.

The ability to restore a protein's expression ("gene addition") or edit one's genetic code ("gene editing") to correct such disease-causing mutations has long been heralded as a transformative medical treatment with the potential to cure a multitude of genetic conditions, such as spinal muscular atrophy, recessive dystrophic epidermolysis bullosa, and GM1 gangliosidosis. However, a substantial challenge to the broad spectrum uses of gene therapy as a first in line treatment strategy remains. The delivery of a gene by itself to a patient's cells is not possible, due to physicochemical barriers preventing entry of nucleic acids into cells.

When used as transection vectors for gene delivery, conventional inactivated virus vectors introduce substantial safety concerns, such as those regarding immunogenicity and insertional mutagenesis. Moreover, they often have high production costs. As a result, treatment systems constructed with non-viral vectors are becoming more promising candidates.

Polymer vectors have been widely studied due to their easy modification and large-scale production ability. In particular, treatments offered by cationic polymer complexes (polyplexes) which are complexes of cationic polymer(s) and nucleic acid(s), which have the advantages of stable structure, easy mass production, easy modification, and increased stability compared to cationic liposomal DNA complexes (lipoplexes).

Since their introduction in 2001 (US8557231B2) and 2016 (WO2016020474A1) respectively, polyplexes comprising linear and hyperbranched poly (β-amino ester)s (LPAEs and HPAEs) have demonstrated potent *in vitro* and *in vivo* gene transfection efficacy and as such have been considered one of the most promising polymeric gene delivery materials for the development of gene therapy medicaments and related medicines. However, such polyplexes are known to lack *in vivo* stability, have low salt stability, high levels of interaction with body fluid components, high uptake by the reticuloendothelial system, and short circulation time, all of which make them unstable.

However, while the leading LPAE- and HPAE-based polyplex candidates generally demonstrate potent efficacy for *in vitro* transfection, these polyplexes can achieve potent *in vivo* delivery efficacy when applied to areas where they can have direct contact with cell tissue, for example through topical application to a wound surface or application to the lung surface *via* inhalation. The stability of these polyplexes in the blood is often low, with polyplexes aggregating or quickly losing efficacy, possibly due to non-specific interactions with blood/body fluid components or vascular walls. This means that several common application methods such as intramuscular or intravenous injection are often unsuitable.

For example, although LPAE- and HPAE-based polyplexes demonstrated potent efficacy when delivering genetic materials to skin tissues *via* intradermal injection/topical application and to lung tissues *via* inhalation or intratracheal instillation, other administration methods, such as systemic or intramuscular administration of such polyplexes failed to generate positive results.

Given the potential indicated by the success of LPAE and HPAE polyplexes in *in vitro* transfection to some tissues, there remains a need for LPAE- and HPAE-based polyplexes which can achieve high transfection efficiency *in vivo,* including *via* challenging delivery methods. The present invention aims to address this issue.

### Summary of the Invention

In one aspect, the invention provides a novel method for the PEG modification of HPAEs, comprising the steps of:
(a) providing a hyperbranched poly(β-amino ester) (HPAE);
(b) mixing the hyperbranched polymer with a poly(ethylene glycol) monomer in a solvent;
(c) reacting the resulting mixture to provide a PEG-modified HPAE;
wherein the level of PEG modification of the PEG-modified HPAE is controlled by one or more of the following parameters:
(i) the reaction temperature;
(ii) the reaction time;
(iii) the feeding ratio of HPAE and PEG monomer;
(iv) the concentration of the reactants in the mixture.

The level of PEG modification of the PEG-modified HPAE may be controlled by selection of the reaction conditions. In one embodiment, the level of PEG modification may be controlled by altering the feeding ratio of HPAE and PEG monomer. Alternatively or additionally, the level of PEG modification may be controlled by altering the reaction time. Alternatively or additionally, the level of PEG modification may be controlled by altering the reaction temperatures. Alternatively or additionally, the level of PEG modification may be controlled by altering the reaction concentration. Of course, the level of PEG modification is also dependent on the HPAE used and the number of functionalisation sites available, for example the number of amine terminals available.

Methods of the invention may lead to the selective formation of PEG-modified HPAEs with varying levels of PEG modification. In some embodiments, the PEG-modified HPAE may have from about 1 to about 30, suitably from about 2 to about 10, preferably 3 or 4 or 5 or 6 or 7 or 8, poly(ethylene glycol) substituents on every repeating HPAE unit.

Suitably, the reaction is carried out at 20 to 30°C, optionally 25 °C. This may achieve a level of PEG modification of up to about 3 PEG substituents per repeating HPAE unit. The a reaction time may be about 15 to 20 h, preferably 18 h, with an HPAE concentration of about 10 mg/mL, and an HPAE:PEG weight feeding ratio of about 2:1. However, altering the reaction temperature may allow the user to control the level of PEG modification.

In another embodiment, the equivalent reaction may be carried out at a temperature of from about 45 to about 55 °C in order to achieve a product having up to about 8 PEG substituents per repeating HPAE unit. Alternatively, the reaction may be carried out at a temperature of from about 75 to about 85 °C in order to achieve up to about 14 PEG substituents per repeating HPAE unit.

Suitably, the reaction components are mixed for a reaction time of about 18 hours. This may achieve a level of PEG modification of up to about 3 PEG substituents per repeating HPAE unit. The reaction temperature may be about 25 °C, with an HPAE concentration of about 10 mg/mL, and an HPAE:PEG weight feeding ratio of about 2:1. However, altering the reaction time may allow the user to control the level of PEG modification.

For example, the equivalent reaction components may be mixed for a reaction time of from about 30 to about 40 hours, such as 36 h, in order to achieve a product having up to about 4 PEG substituents per repeating HPAE unit. Alternatively, the reaction components may be mixed for a reaction time of from about 40 to about 50 hours, such as 48 h, in order to achieve a product having up to about 5 PEG substituents per repeating HPAE unit.

Suitably, the reaction is carried out at a concentration of HPAE in the reaction mixture of about 10 mg/mL. This may achieve a level of PEG modification of up to about 3 PEG substituents per repeating HPAE unit. The reaction time may be about 18 h, with a reaction temperature of about 25 °C, and an HPAE:PEG weight feeding ratio of about 2:1. However, altering the reaction concentration may allow the user to control the level of PEG modification.

For example, the equivalent reaction may be carried out at an HPAE concentration of from about 25 to about 35 mg/mL, such as about 30 mg/mL, in order to achieve a product having up to about 5 PEG substituents per repeating HPAE unit. Alternatively, the reaction may be carried out at an HPAE concentration of from about 45 to about 55 mg/mL, such as about 50 mg/mL, in order to achieve up to about 6 PEG substituents per repeating HPAE unit.

Suitably, the reaction components are mixed with an HPAE:PEG weight feed ratio of about 2:1. This may achieve a level of PEG modification of up to about 3 PEG substituents per repeating HPAE unit. The reaction time may be about 18 h, with a reaction temperature of about 25 °C, an HPAE concentration of about 10 mg/mL. However, altering the feeding ratio may allow the user to control the level of PEG modification.

For example, the equivalent reaction may be carried out at an HPAE:PEG weight feed ratio of from about 1:4 to about 1:6, such as about 1:5, in order to achieve a product having up to about 6 PEG substituents per repeating HPAE unit. Alternatively, the reaction may be carried out at an HPAE:PEG weight feed ratio of from about 1:8 to about 1:12, such as about 1:10, in order to achieve a product having up to about 10 PEG substituents per repeating HPAE unit.
The hyperbranched polymer may be an HPAE as described in WO2021058491A1, which is incorporated herein by reference.

For example, the hyperbranched polymer may be an HPAE made by reacting together:
(i) at least one four-branching monomer having four reaction sites that can react with acrylate or amine groups;
(ii) at least one diacrylate component, typically of formula (I) 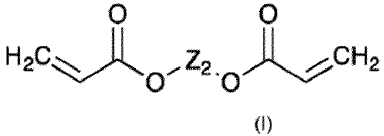 wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms; wherein Z2 is unsubstituted or substituted with at least one of a halogen, a hydroxyl, an amino group, a sulfonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(O)NR'R', -N(R')C(O)NR'R, - N(R')C(O)O-C1-C6 alkyl, C3-C6 cycloalkyl, C3-C6 heterocyclyl, C2-C5 heteroaryl and C6-C10 aryl; wherein each R' is independently selected from the group consisting of hydrogen and C1-C6 alkyl;
(iii) at least one first amine component typically comprising 3 to 20 carbon atoms, wherein said at least one first amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(O)NR'R', -N(R')C(O)NR'R, -N(R')C(O)O-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(iv) at least one second amine component typically comprising 3 to 20 carbon atoms, wherein said at least one second amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(O)NR'R', - N(R')C(O)NR'R, -N(R')C(O)O-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.

The hyperbranched polymer may be an HPAE formed using an oligomer combination approach, in which the diacrylate and first amine components are reacted together to form a first oligomer, the first oligomer and second amine component are reacted together to form a second oligomer, and the second oligomer and four branching monomer are reacted together to form an HPAE. This oligomer combination approach is described in detail in Zeng et al (Nano. Lett. 2019 19, 381-391).

Alternatively, the hyperbranched polymer may be an HPAE formed by reacting the four-branching monomer, diacrylate component, and first amine together in a Michael addition reaction to form a first polymer, and then reacting the first polymer and second amine component (endcapping amine together in a Michael addition reaction to form the HPAE.

The use of a four-branching monomer leads to an HPAE polymer with multiple sites available for PEG functionalisation.

The at least one four-branching monomer may suitably be a diamine component, or a tetraacrylate component, although other four-branching monomers may be employed such as for example any component with tetra acrylamide groups (i.e. 4-arm PEG acrylamide, 4-arm PEG maleimide), any component with tetra N-hydroxysuccinimide (NHS) groups (i.e. 4-arm PEG-succinimidyl carbonate NHS ester), any type of tetrathiol component (i.e. Pentaerythritol tetrakis(3- mercaptopropionate), 4-arm PEG-thiol, Tetra(2-mercaptoethyl)silane), and any tetraepoxy component (i.e. TetraGlycidyl methylenedianiline, Tetraglycidyl 1,1'- methylenebis(naphthalene-2,7-diol), Pentaerythritol tetraglycidyl ether, 4-arm peg epoxide).

The at least one four-branching monomer may be selected from the group comprising:

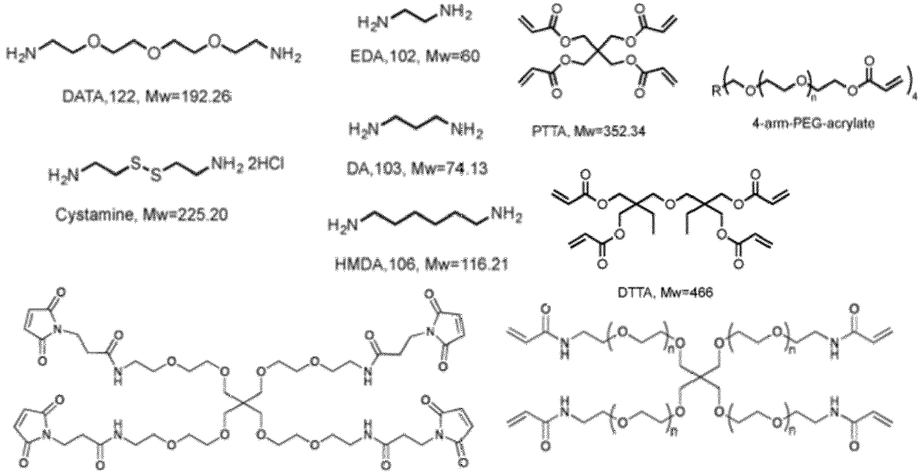

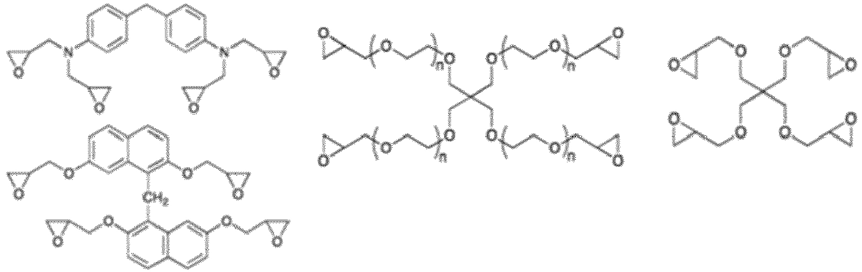

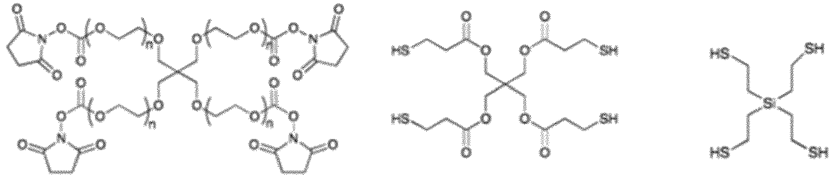

Suitably, the four-branching monomer may be PTTA:

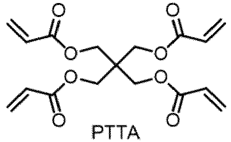

In other words, the hyperbranched polymer may be an HPAE which is a derivative of one or more of the above monomers, suitably pentaerythritol tetraacrylate (PTTA), as a branching monomer.

The at least one diacrylate component may be selected from the group comprising:

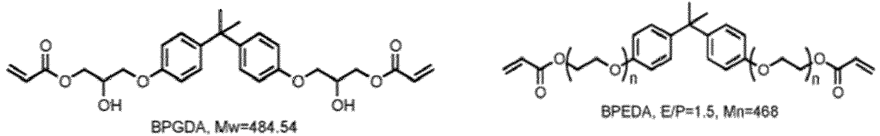

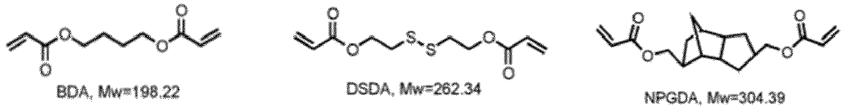

Suitably, the at least one diacrylate component may be selected from the group comprising: 1,4-butanediol diacrylate (B4); 1,5-pentanediol diacrylate (B5).

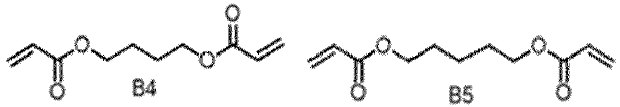

In other words, the hyperbranched polymer may be an HPAE which is a derivative of one or more of the above diacrylates, suitably B4 or B5, as a backbone monomer.

The at least one first amine component may be selected from the group comprising:

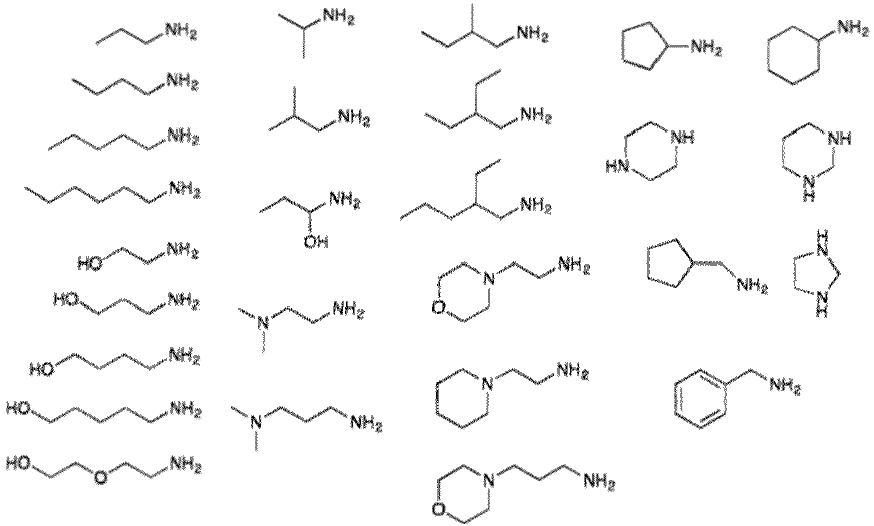

Suitably, the at least one first amine component may be selected from the group comprising: 6-amino-1-hexanol (S6); 5-amino-1-pentanol (S5).

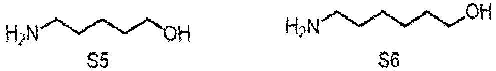

In other words, the hyperbranched polymer may be an HPAE which is a derivative of one or more of the above amines, suitably S5 or S6, as a backbone monomer.

The at least one second amine component may be a diamine component having a structure NH₂-L₄-NH₂ or may be selected from the group comprising:

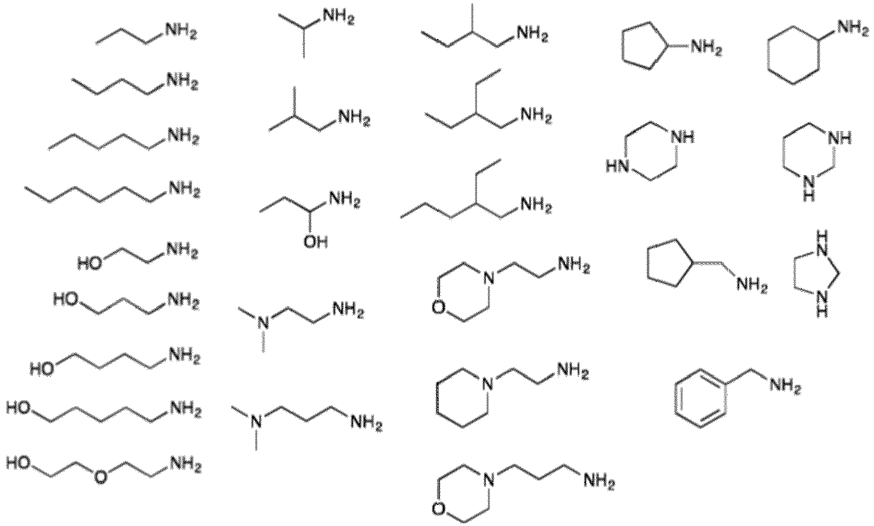

Suitably, the at least one second amine component is selected from the group comprising:

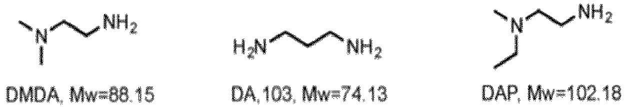

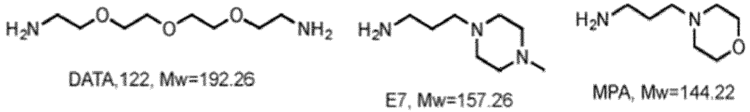

Preferably, the at least one second amine component is selected from the group comprising: 1,2-diaminoethane (102); 1,3-diaminopropane (103); 1,11-diamino-3,6,9-trioxaundecane (122); 1-(3-aminopropyl)-4-methylpiperazine (E7).

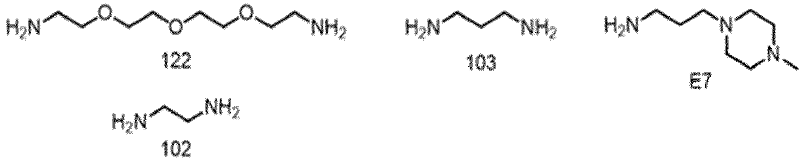

In other words, the hyperbranched polymer may be an HPAE which is a derivative of one or more of the above amines, preferably E7, 102, 103, or 122, as an endcapping monomer.

Suitably, the hyperbranched polymer is an HPAE which is a derivative of one of the following combinations of monomers:

| **Ref.** | **Four-branching monomer** | **Diacrylate component** | **First amine component (backbone amine)** | **Second amine component (endcapping amine)** |
|---|---|---|---|---|
| **1** | 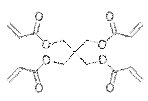 | 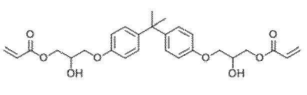 | 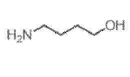 | 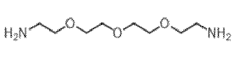 |
| **2** | 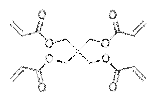 | 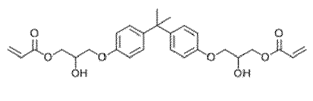 | 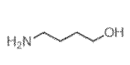 | 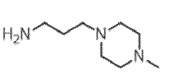 |
| **3** | 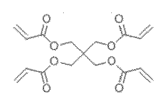 | 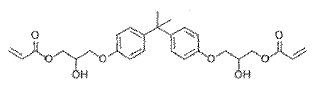 | 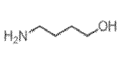 | 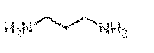 |
| **4** | 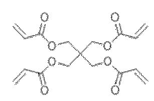 | 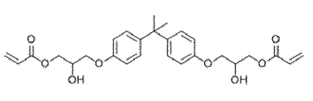 | 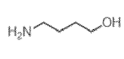 | 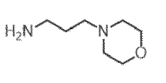 |
| **5** | 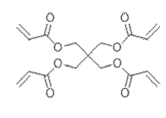 | 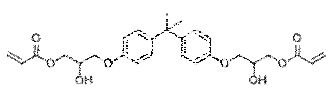 | 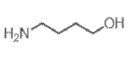 | 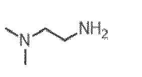 |
| **6** | 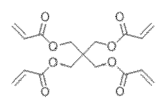 | 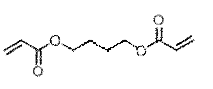 | 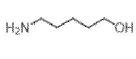 | 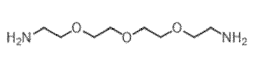 |
| **7** | 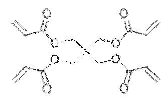 | 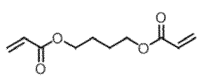 | 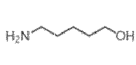 | 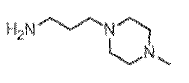 |
| **8** | 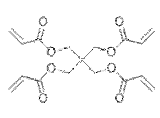 | 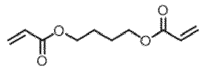 | 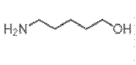 | 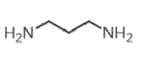 |
| | | | | |
| **9** | 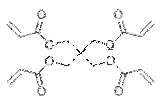 | 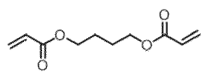 | 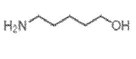 | 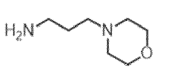 |
| **10** | 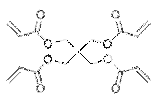 | 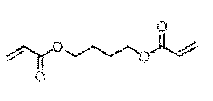 | 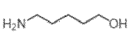 | 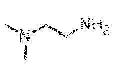 |
| **11** | 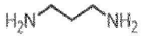 | 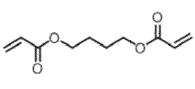 | 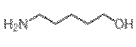 | 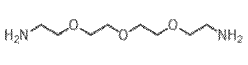 |
| **12** | 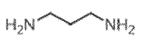 | 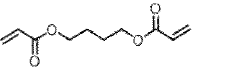 | 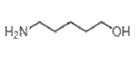 | 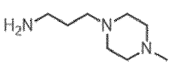 |
| **13** | 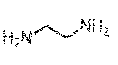 | 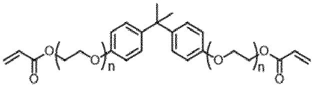 | 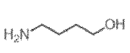 | 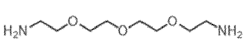 |
| 1**4** | 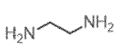 | 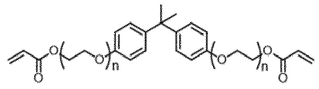 | 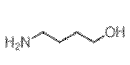 | 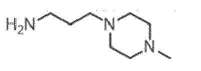 |
| | | | | |
| **15** | 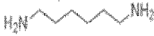 | 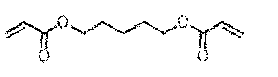 | 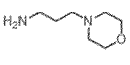 | 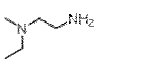 |
| **16** | 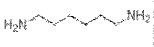 | 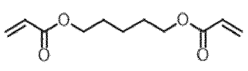 | 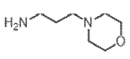 | 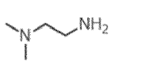 |
| **17** | 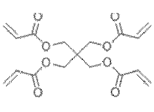 | 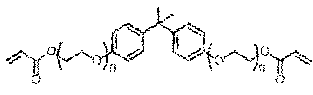 | 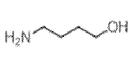 | 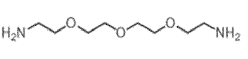 |
| **18** | 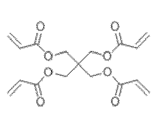 | 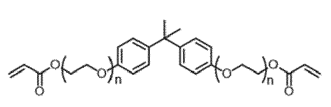 | 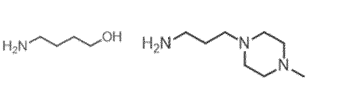 | |
| **19** | 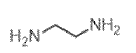 | 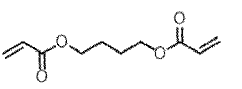 | 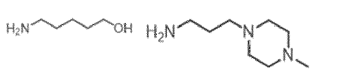 | |
| **20** | 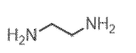 | 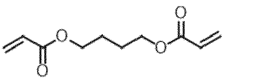 | 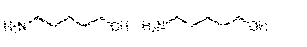 | |

Desirably, the hyperbranched polymer may be an HPAE which is a derivative of one of the monomer combinations: 1, 4, 6, 8, 12, 14, 16, or 19.

Preferably, the hyperbranched polymer may be an HPAE which is a derivative of S5 or S6 as an amine backbone monomer, B5 or B4 as a diacrylate monomer, PTTA as a branching monomer, and 122 as an endcapping monomer.

Suitably, the hyperbranched polymer is an HPAE having a molecular weight Mw of from about 10 to about 80 kDa, suitably from about 10 to about 40 kDa, preferably from about 15-25 kDA.

The hyperbranched polymer may be an HPAE having an average of from about 5 to about 80, suitably from about 15 to about 40 amine terminals per polymer molecule, preferably an average of from about 10 to about 25 amine terminals, some or all of which may serve as sites for PEG functionalisation.

It is believed that the multiple terminals available on HPAEs allow for a wider variety of levels of PEG modification in comparison to linear poly(β-amino ester)s (LPAEs).

The poly(ethylene glycol) monomer may be selected from the list comprising: a poly(ethylene glycol) acrylate; a poly(ethylene glycol) ether acrylate; a poly(ethylene glycol) methyl acrylate; a poly(ethylene glycol) acrylamide; a poly(ethylene glycol) epoxide; a poly(ethylene glycol) *N*-hydroxysuccinimide; a poly(ethylene glycol) carboxyl; a poly(ethylene glycol) vinyl sulfone; a poly(ethylene glycol) maleimide.

Suitably, the poly(ethylene glycol) monomer is a poly(ethylene glycol) acrylate or a poly(ethylene glycol) ether acrylate.

Preferably, the poly(ethylene glycol) monomer is a poly(ethylene glycol) methyl ether acrylate with an average molecular weight Mₙ of from about 400 Da to about 10,000 Da, such as from about 1500 to about 2000 Da. Examples may include poly(ethylene glycol) methyl ether acrylates having an average molecular weight Mₙ of about 480, 2000, or 5000 Da.

The hyperbranched polymer and the poly(ethylene glycol) may be mixed in a weight ratio of from about 1:20 to about 10:1, suitably from about 1:10 to about 5:1, preferably from about 1:5 to about 3:1.

In another aspect, the invention relates to a PEG-modified HPAE as described above. The PEG-modified hyperbranched poly(β-amino ester) (HPAE) is an HPAE which has been modified to have one or more poly(ethylene glycol) (PEG) functional groups, optionally via a method as described herein.

In another aspect, the present invention relates to a polyplex of a nucleic acid and a PEG-modified hyperbranched poly(β-amino ester) (HPAE).

The PEG-modified hyperbranched poly(β-amino ester) (HPAE) may be any HPAE which has been modified to have one or more poly(ethylene glycol) (PEG) functional groups, optionally via a method as described herein.

The nucleic acid may be DNA. Alternatively the nucleic acid may be RNA, for example mRNA. Where the nucleic acid used is DNA, the resulting polyplex may be suitable for delivering DNA to a cell. Where the nucleic acid used is RNA, the resulting polyplex may be suitable for delivering RNA to a cell.

The nucleic acid and the PEG-modified HPAE may be present in the polyplex in a weight ratio of from about 1:1 to about 1:200 respectively, suitably 1:5 to about 1:50 respectively, preferably from about 1:10 to about 1:40 respectively. The nucleic acid and the PEG-modified HPAE may be present in the polyplex in a ratio of about 1:20 or about 1:10 respectively.

The PEG-modified HPAE may be the only polymer present in the polyplex. Where this is the case, it is desirable that the PEG-modified HPAE has fewer than about 15 PEG substituents on every repeating HPAE unit, suitably from 1 to about 10, preferably from 1 to about 4. Without wishing to be bound by theory, it is believed that this relatively low level of PEG substitution achieves sufficient *in vivo* stability without compromising on particle stability or nucleic acid binding efficiency.

Alternatively, the polyplex may comprise a second cationic polymer.

Where this is the case, a polyplex of the invention may have a core-shell structure, wherein the polyplex has a shell comprising PEG-modified HPAE and a core comprising the nucleic acid and a second cationic polymer.

Preferably, the second cationic polymer has no PEG functionalisation. In embodiments where a second cationic polymer without PEG functionalisation is present, the PEG-modified HPAE may have from about 5 to about 60 PEG substituents on every repeating HPAE unit, suitably from about 5 to about 30, or from about 10 to about 20.

Without wishing to be bound by theory, it is believed that the presence of PEG functional groups in the shell but not in the core imparts high *in vivo* stability to the polyplex while maintaining a condensed core with high thermostability which is not affected by PEG functionality. Preferably, the PEG-modified HPAE is entirely located within the shell of the polyplex.

The second cationic polymer may be another PAE. Suitably, it may be a linear poly(β-amino ester)s (LPAE) or a hyperbranched poly(β-amino ester) (HPAE). Preferably, the second cationic polymer is an LPAE or HPAE without PEG modification.

Preferably, a polyplex of the invention has a core comprising the nucleic acid and an LPAE or HPAE without PEG modification and a shell comprising a PEG-modified HPAE. While the nucleic acid is expected to be predominantly present in the core of the polyplex, it may also be present at the interface between the core and the shell and potentially could be present in smaller amounts in the shell of the polyplex.

The PEG-modified HPAE and the second cationic polymer may be present in the polyplex in a weight ratio of from about 5:1 to about 1:100 respectively, such as from about 1:1 to about 1:20 respectively, for example from about 1:2 to about 1:10 respectively, for example about 1:2 respectively or about 1:5 respectively.

Without wishing to be bound by theory, it is believed that polyplex formation is achieved through the interaction between the electronegative carbonyl oxygen in the ester group and the cationic amine in the terminal and backbone of LPAEs or HPAEs. In the present invention, it is believed that when the shell of the polyplex comprises a PEG-modified HPAE, both the amine terminals and the PEG terminals are able to complex readily with a core comprising non-PEG-modified HPAEs or LPAEs. It is further believed that this effect is not achieved with PEG-modified LPAEs, which lack terminal amine groups and which have fewer backbone amine moieties than their hyperbranched counterparts.

A polyplex of the invention may further comprise additional compounds, which may be selected, for example, for formulation purposes or for increased stability. Suitably, a polyplex of the invention comprises one or more compounds selected from the following: small molecular amines, cholestrol, neutral lipids (for example DOPE or DSPC), targeting or membrane fusion peptides.

A polyplex of the invention may be suitable for use as a vector for transfection. In particular, it may be suitable for use in *in vivo* transfection.

The invention also provides a method of using a polyplex as described herein as a vector for transfection, such as a vector for in vivo transfection, in the treatment of disease, such as spinal muscular atrophy, recessive dystrophic epidermolysis bullosa and GM1 gangliosidosis, as well as for vaccines against infectious diseases or cancers.

Without wishing to be bound by theory, it is believed that the presence of a PEG substituent on the HPAE may increase the resulting polyplexes' salt stability, reduce their interaction with body fluid components such as proteins, hemocytes, leukocytes and other salts, decrease their uptake by the reticuloendothelial system and prolong their circulation time in the bloodstream when compared to polyplexes prepared from HPAEs without PEG modification. PEG functionalisation is believed to improve particle pharmacokinetic behavior, which can shield the surface from aggregation, opsonisation and phagocytosis, reduce non-specific interactions with serum components, and prolong systemic circulation. These effects may all contribute to improved *in vivo* transfection efficiency of the polyplexes of the invention.

Furthermore, it is believed that the use of HPAEs with PEG modification may also inhibit nucleic acid hydrolysis, reduce degradation speeds of the polymer(s) in the polyplex and help the polyplexes to retain full *in vivo* efficacy even after lyophilisation.

When used to transfect HEK293 cells, a polyplex of the invention may achieve a percentage of transfected HEK293 cells of greater than about 90% when tested using flow cytometry. The transfection efficiency for other cell lines may differ. Flow cytometry methods are described herein.

While not wishing to be bound by theory, it is believed that polyplexes of the invention may have cores which are smaller and more uniformly sized than those created by previously known methods, and this may lead to the polyplexes of the invention achieving high transfection efficiencies.

A polyplex of the invention may be stable for at least 24 hours, suitably at least 8 hours, in aqueous solution at 37 °C.

In another aspect the invention provides a method for producing a polyplex of a nucleic acid and PEG-modified hyperbranched poly(β-amino ester) comprising the step of mixing a nucleic acid with a PEG-modified hyperbranched poly(β-amino ester).

The nucleic acid and the PEG-modified HPAE may be mixed in a weight ratio of from about 1:5 to about 1:50 respectively, suitably from about 1:10 to about 1:40 respectively, preferably from about 1:15 to about 1:30 respectively. The nucleic acid and the PEG-modified HPAE may be mixed in a ratio of about 1:20 or about 1:10 respectively.

The mixing may be carried out in an ionic buffer, such as a sodium acetate buffer. For example, the nucleic acid, the PEG-modified HPAE, or both may be in a solution of ionic buffer.

The nucleic acid used in a method of the invention may be introduced in isolated form, with or without the presence of a buffer and/or a solvent. Alternatively, the nucleic acid may be introduced as part of a polyplex core with a second cationic polymer.

The method of the invention may therefore comprise the additional earlier step of mixing the nucleic acid with a second cationic polymer to form a core, which can then be mixed with the PEG-modified HPAE.

The step of mixing the nucleic acid with a second cationic polymer to form a core may be carried out in an ionic buffer, such as a sodium acetate buffer. For example, the nucleic acid, the second cationic polymer, or both may be in a solution of ionic buffer.

The nucleic acid and the second cationic polymer may be mixed in a weight ratio of from about 5:1 to about 1:200 respectively, such as from about 1:1 to about 1:20 respectively, for example from about 1:2 to about 1:10 respectively, for example about 1:5 respectively or about 1:10 respectively.

The core may then be mixed with the PEG-modified HPAE in a weight ratio of from about 0:1 to about 100:1 respectively.

In another aspect the invention also provides novel PEG-modified HPAEs and a method for producing same.

### Definitions

Given below are the condensed (by no means exhaustive) customary definitions known in the art, of which certain terms which may aid in the description of the invention.

'Polyplex' refers to a complex of cationic polymer and nucleic acid. A polyplex may be used to deliver exogenous DNA or RNA to cells through transfection.

'Cargo' refers to the portion of nucleic acid which is present in the polyplex and which can be delivered to cells by transfection. The cargo is believed to be held in the polyplex by entanglement with the cationic polymer(s).

'Core' refers to a small local aggregation formed between the nucleic acid and the first cationic polymer.

'Single-polymer polyplex' refers to a polyplex having been prepared from only one cationic polymer. In embodiments of the invention, a single-polymer polyplex comprises a PEG-modified HPAE but does not comprise a second cationic polymer. Non-inventive embodiments, comprising a non-PEGylated HPAE and no second cationic polymer, have also been described for comparison.

'Core-shell functional polyplex' refers to a polyplex of the invention comprising a PEG-functionalised HPAE, which forms the shell of the polyplex, and a second HPAE without PEG functionalisation which, along with the nucleic acid cargo, forms the core of the polyplex.

### Brief Description of the Drawings

Embodiments of the invention will be described, by way of example only, with reference to the accompanying drawings in which:
**Figure 1** shows normalised luminescence values measured 48 h after transfection of HEK293 with single-polymer polyplexes of the invention;
**Figure 2** shows a graph of polyplex size for polyplexes of the invention both with and without dilution in buffer solution(s), indicating their salt stability;
**Figure 3** shows the results of a turbidity assay for polyplexes of the invention after dilution in buffer solution(s), indicating their serum stability.
**Figure 4** shows normalised luminescence values measured 48 h after transfection of HEK293 with core-shell polyplexes of the invention;
**Figure 5** shows normalised luminescence values measured after transfection of HEK293 with core-shell polyplexes of the invention, including those in solution and those having been lyophilised, after storage at various temperatures over a series of time points, in order to demonstrate the stability of the polyplexes.
**Figure 6** shows the *in vivo* transfection ability of fresh, frozen, and lyophilised polyplexes of the invention administered intravenously in mice.
**Figure 7** shows the size distribution of core-shell functional polyplexes made with different levels of PEG modification.

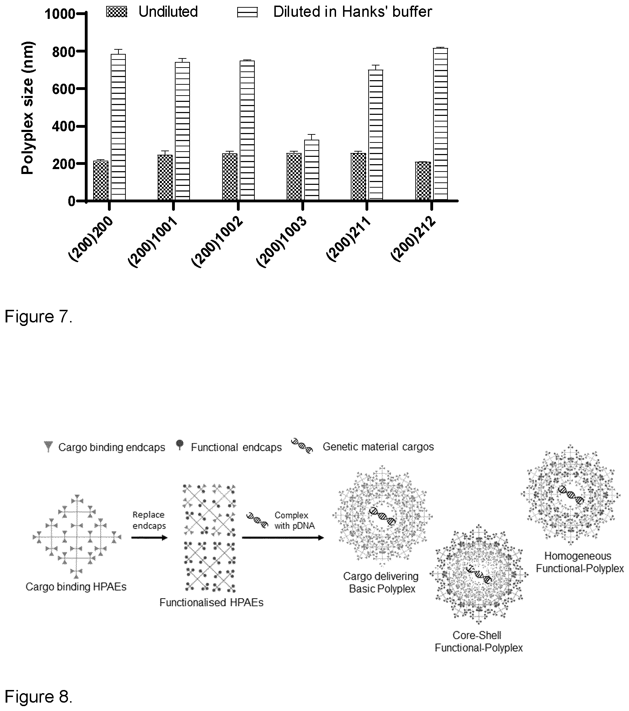

**Figure 8** shows a representation of a pathway to a range of polyplexes with different structures. The 'cargo delivering basic polyplex' represents a 'traditional' polyplex, made with a single HPAE polymer and having a nucleic acid (in this case pDNA) cargo. The remaining two polyplex structures represent polyplexes of the invention. The 'homogeneous functional polyplex' comprises a PEG-functionalised HPAE and a nucleic acid cargo. The 'core-shell functional polyplex' comprises a PEG-functionalised HPAE, which forms the shell of the polyplex, and a second HPAE without PEG functionalisation which, along with the nucleic acid cargo, forms the core of the polyplex. In this core-shell polyplex, it is believed that the presence of PEG functionality only in the shell portion of the polyplex keeps the functionalised endcaps at the polyplex surface for optimal clinical interactions, while reducing the risk of any potential interruptions which may arise between the nucleic acid and the polymer carrier(s) and which could reduce polyplex thermostability.

### Detailed Description of the Drawings

A selection of exemplary backbone monomers and endcapping monomers, as shown below, were used to prepare various HPAEs as described in WO2021058491A1. The HPAEs were then reacted with commercially available poly(ethylene glycol) methyl ether acrylates with various average molecular weights Mn, in order to generate a library of PEG-modified HPAEs as shown in Table 1.

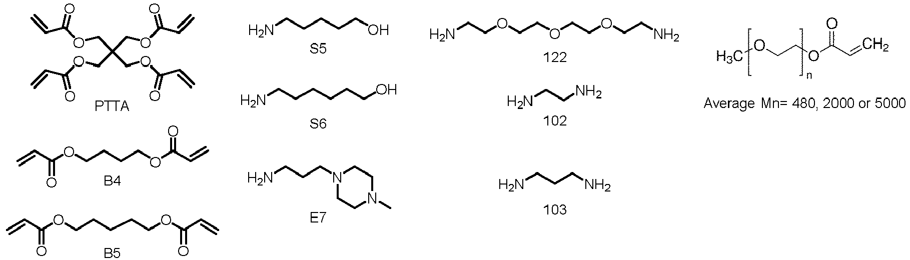

In order to synthesise the HPAEs, the backbone monomers were weighed and dissolved in DMSO to form a solution with the appropriate ratio of monomer concentrations. The mixture was placed in an oil bath at 90 °C for polymerisation. When the desired molecular weight was reached, the reaction mixture was diluted with DMSO and the appropriate endcap monomer was added. The endcapping reaction was then carried out at room temperature. The crude reaction mixture was purified with a diethyl ether wash before being collected and dried under a vacuum. A portion of the product was dissolved in DMSO at a concentration of 100 mg/mL for direct use as a non-PEG-modified HPAE. The remainder was mixed with a DMSO solution of the appropriate molecular weight poly(ethylene glycol) methyl ether acrylate in the desired weight ratio and reacted to form the PEG-modified HPAEs listed in Table 1.

**Table 1 - The library of HPAEs prepared for testing**

| **Ref.** | **Amine monomer** | **Diacrylate monomer** | **Branching monomer** | **Endcap monomer** | **Average Mn of PEG acrylate** | **Weight ratio PEG acrylate : HPAE** |
|---|---|---|---|---|---|---|
| **0** | S6 | B5 | PTTA | 122 | N/A | N/A |
| **1** | S6 | B5 | PTTA | 122 | 480 | 1:12 |
| **2** | S6 | B5 | PTTA | 122 | 480 | 1:8 |
| **3** | S6 | B5 | PTTA | 122 | 480 | 1:4 |
| **4** | S6 | B5 | PTTA | 122 | 480 | 1:2 |
| **5** | S6 | B5 | PTTA | 122 | 480 | 3:4 |
| 6 | S6 | B5 | PTTA | 122 | 480 | 5:4 |
| **7** | S6 | B5 | PTTA | 122 | 2000 | 1:3 |
| **8** | S6 | B5 | PTTA | 122 | 2000 | 1:2 |
| 9 | S6 | B5 | PTTA | 122 | 2000 | 1:1 |
| **10** | S6 | B5 | PTTA | 122 | 2000 | 2:1 |
| **11** | S6 | B5 | PTTA | 122 | 2000 | 3:1 |
| **12** | S6 | B5 | PTTA | 122 | 2000 | 5:1 |
| **13** | S6 | B5 | PTTA | 122 | 5000 | 5:6 |
| **14** | S6 | B5 | PTTA | 122 | 5000 | 5:4 |
| **15** | S6 | B5 | PTTA | 122 | 5000 | 5:2 |
| **16** | S6 | B5 | PTTA | 122 | 5000 | 5:1 |
| **17** | S6 | B5 | PTTA | 122 | 5000 | 15:2 |
| **18** | S6 | B5 | PTTA | 122 | 5000 | 25:2 |
| **50** | S5 | B4 | PTTA | 122 | NA | NA |
| **51** | S5 | B4 | PTTA | 122 | 480 | 1:12 |
| **52** | S5 | B4 | PTTA | 122 | 480 | 1:8 |
| **53** | S5 | B4 | PTTA | 122 | 480 | 1:4 |
| **54** | S5 | B4 | PTTA | 122 | 480 | 1:2 |
| **55** | S5 | B4 | PTTA | 122 | 480 | 3:4 |
| **56** | S5 | B4 | PTTA | 122 | 480 | 5:4 |
| **57** | S5 | B4 | PTTA | 122 | 2000 | 1:3 |
| **58** | S5 | B4 | PTTA | 122 | 2000 | 1:2 |
| **59** | S5 | B4 | PTTA | 122 | 2000 | 1:1 |
| **60** | S5 | B4 | PTTA | 122 | 2000 | 2:1 |
| **61** | S5 | B4 | PTTA | 122 | 2000 | 3:1 |
| **62** | S5 | B4 | PTTA | 122 | 2000 | 5:1 |
| **63** | S5 | B4 | PTTA | 122 | 5000 | 5:6 |
| **64** | S5 | B4 | PTTA | 122 | 5000 | 5:4 |
| **65** | S5 | B4 | PTTA | 122 | 5000 | 5:2 |
| **66** | S5 | B4 | PTTA | 122 | 5000 | 5:1 |
| **67** | S5 | B4 | PTTA | 122 | 5000 | 15:2 |
| **68** | S5 | B4 | PTTA | 122 | 5000 | 25:2 |
| **100** | S6 | B5 | PTTA | 102 | NA | NA |
| **101** | S6 | B5 | PTTA | 102 | 480 | 1:12 |
| **102** | S6 | B5 | PTTA | 102 | 480 | 1:8 |
| **103** | S6 | B5 | PTTA | 102 | 480 | 1:4 |
| **104** | S6 | B5 | PTTA | 102 | 480 | 1:2 |
| **105** | S6 | B5 | PTTA | 102 | 480 | 3:4 |
| **106** | S6 | B5 | PTTA | 102 | 480 | 5:4 |
| **107** | S6 | B5 | PTTA | 102 | 2000 | 1:3 |
| **108** | S6 | B5 | PTTA | 102 | 2000 | 1:2 |
| **109** | S6 | B5 | PTTA | 102 | 2000 | 1:1 |
| **110** | S6 | B5 | PTTA | 102 | 2000 | 2:1 |
| **111** | S6 | B5 | PTTA | 102 | 2000 | 3:1 |
| **112** | S6 | B5 | PTTA | 102 | 2000 | 5:1 |
| **113** | S6 | B5 | PTTA | 102 | 5000 | 5:6 |
| **114** | S6 | B5 | PTTA | 102 | 5000 | 5:4 |
| **115** | S6 | B5 | PTTA | 102 | 5000 | 5:2 |
| **116** | S6 | B5 | PTTA | 102 | 5000 | 5:1 |
| **117** | S6 | B5 | PTTA | 102 | 5000 | 15:2 |
| **118** | S6 | B5 | PTTA | 102 | 5000 | 25:2 |
| **200** | S5 | B4 | PTTA | 102 | NA | NA |
| **201** | S5 | B4 | PTTA | 102 | 480 | 1:12 |
| **202** | S5 | B4 | PTTA | 102 | 480 | 1:8 |
| **203** | S5 | B4 | PTTA | 102 | 480 | 1:4 |
| **204** | S5 | B4 | PTTA | 102 | 480 | 1:2 |
| **205** | S5 | B4 | PTTA | 102 | 480 | 3:4 |
| **206** | S5 | B4 | PTTA | 102 | 480 | 5:4 |
| **207** | S5 | B4 | PTTA | 102 | 2000 | 1:3 |
| **208** | S5 | B4 | PTTA | 102 | 2000 | 1:2 |
| **209** | S5 | B4 | PTTA | 102 | 2000 | 1:1 |
| **210** | S5 | B4 | PTTA | 102 | 2000 | 2:1 |
| **211** | S5 | B4 | PTTA | 102 | 2000 | 3:1 |
| **212** | S5 | B4 | PTTA | 102 | 2000 | 5:1 |
| **213** | S5 | B4 | PTTA | 102 | 5000 | 5:6 |
| **214** | S5 | B4 | PTTA | 102 | 5000 | 5:4 |
| **215** | S5 | B4 | PTTA | 102 | 5000 | 5:2 |
| **216** | S5 | B4 | PTTA | 102 | 5000 | 5:1 |
| **217** | S5 | B4 | PTTA | 102 | 5000 | 15:2 |
| **218** | S5 | B4 | PTTA | 102 | 5000 | 25:2 |
| **500** | S6 | B5 | PTTA | E7 | NA | NA |
| **501** | S6 | B5 | PTTA | E7 | 480 | 1:12 |
| **502** | S6 | B5 | PTTA | E7 | 480 | 1:8 |
| **503** | S6 | B5 | PTTA | E7 | 480 | 1:4 |
| **504** | S6 | B5 | PTTA | E7 | 480 | 1:2 |
| **505** | S6 | B5 | PTTA | E7 | 480 | 3:4 |
| **506** | S6 | B5 | PTTA | E7 | 480 | 5:4 |
| **507** | S6 | B5 | PTTA | E7 | 2000 | 1:3 |
| **508** | S6 | B5 | PTTA | E7 | 2000 | 1:2 |
| **509** | S6 | B5 | PTTA | E7 | 2000 | 1:1 |
| **510** | S6 | B5 | PTTA | E7 | 2000 | 2:1 |
| **511** | S6 | B5 | PTTA | E7 | 2000 | 3:1 |
| **512** | S6 | B5 | PTTA | E7 | 2000 | 5:1 |
| **513** | S6 | B5 | PTTA | E7 | 5000 | 5:6 |
| **514** | S6 | B5 | PTTA | E7 | 5000 | 5:4 |
| **515** | S6 | B5 | PTTA | E7 | 5000 | 5:2 |
| **516** | S6 | B5 | PTTA | E7 | 5000 | 5:1 |
| **517** | S6 | B5 | PTTA | E7 | 5000 | 15:2 |
| **518** | S6 | B5 | PTTA | E7 | 5000 | 25:2 |
| **600** | S5 | B4 | PTTA | E7 | NA | NA |
| **601** | S5 | B4 | PTTA | E7 | 480 | 1:12 |
| **602** | S5 | B4 | PTTA | E7 | 480 | 1:8 |
| **603** | S5 | B4 | PTTA | E7 | 480 | 1:4 |
| **604** | S5 | B4 | PTTA | E7 | 480 | 1:2 |
| **605** | S5 | B4 | PTTA | E7 | 480 | 3:4 |
| **606** | S5 | B4 | PTTA | E7 | 480 | 5:4 |
| **607** | S5 | B4 | PTTA | E7 | 2000 | 1:3 |
| **608** | S5 | B4 | PTTA | E7 | 2000 | 1:2 |
| **609** | S5 | B4 | PTTA | E7 | 2000 | 1:1 |
| **610** | S5 | B4 | PTTA | E7 | 2000 | 2:1 |
| **611** | S5 | B4 | PTTA | E7 | 2000 | 3:1 |
| **612** | S5 | B4 | PTTA | E7 | 2000 | 5:1 |
| **613** | S5 | B4 | PTTA | E7 | 5000 | 5:6 |
| **614** | S5 | B4 | PTTA | E7 | 5000 | 5:4 |
| **615** | S5 | B4 | PTTA | E7 | 5000 | 5:2 |
| **616** | S5 | B4 | PTTA | E7 | 5000 | 5:1 |
| **617** | S5 | B4 | PTTA | E7 | 5000 | 15:2 |
| **618** | S5 | B4 | PTTA | E7 | 5000 | 25:2 |
| 800 | S6 | B5 | PTTA | 103 | NA | NA |
| **801** | S6 | B5 | PTTA | 103 | 480 | 1:12 |
| **802** | S6 | B5 | PTTA | 103 | 480 | 1:8 |
| **803** | S6 | B5 | PTTA | 103 | 480 | 1:4 |
| **804** | S6 | B5 | PTTA | 103 | 480 | 1:2 |
| **805** | S6 | B5 | PTTA | 103 | 480 | 3:4 |
| **806** | S6 | B5 | PTTA | 103 | 480 | 5:4 |
| **807** | S6 | B5 | PTTA | 103 | 2000 | 1:3 |
| **808** | S6 | B5 | PTTA | 103 | 2000 | 1:2 |
| **809** | S6 | B5 | PTTA | 103 | 2000 | 1:1 |
| **810** | S6 | B5 | PTTA | 103 | 2000 | 2:1 |
| **811** | S6 | B5 | PTTA | 103 | 2000 | 3:1 |
| **812** | S6 | B5 | PTTA | 103 | 2000 | 5:1 |
| **813** | S6 | B5 | PTTA | 103 | 5000 | 5:6 |
| **814** | S6 | B5 | PTTA | 103 | 5000 | 5:4 |
| **815** | S6 | B5 | PTTA | 103 | 5000 | 5:2 |
| **816** | S6 | B5 | PTTA | 103 | 5000 | 5:1 |
| **817** | S6 | B5 | PTTA | 103 | 5000 | 15:2 |
| **818** | S6 | B5 | PTTA | 103 | 5000 | 25:2 |
| **900** | S5 | B4 | PTTA | 103 | NA | NA |
| **901** | S5 | B4 | PTTA | 103 | 480 | 1:12 |
| **902** | S5 | B4 | PTTA | 103 | 480 | 1:8 |
| **903** | S5 | B4 | PTTA | 103 | 480 | 1:4 |
| **904** | S5 | B4 | PTTA | 103 | 480 | 1:2 |
| **905** | S5 | B4 | PTTA | 103 | 480 | 3:4 |
| **906** | S5 | B4 | PTTA | 103 | 480 | 5:4 |
| **907** | S5 | B4 | PTTA | 103 | 2000 | 1:3 |
| **908** | S5 | B4 | PTTA | 103 | 2000 | 1:2 |
| **909** | S5 | B4 | PTTA | 103 | 2000 | 1:1 |
| **910** | S5 | B4 | PTTA | 103 | 2000 | 2:1 |
| **911** | S5 | B4 | PTTA | 103 | 2000 | 3:1 |
| **912** | S5 | B4 | PTTA | 103 | 2000 | 5:1 |
| **913** | S5 | B4 | PTTA | 103 | 5000 | 5:6 |
| **914** | S5 | B4 | PTTA | 103 | 5000 | 5:4 |
| **915** | S5 | B4 | PTTA | 103 | 5000 | 5:2 |
| **916** | S5 | B4 | PTTA | 103 | 5000 | 5:1 |
| **917** | S5 | B4 | PTTA | 103 | 5000 | 15:2 |
| **918** | S5 | B4 | PTTA | 103 | 5000 | 25:2 |
| **1001** | S5 | B4 | PTTA | 102 | 480 | 1:60 |
| **1002** | S5 | B4 | PTTA | 102 | 480 | 1:30 |
| **1003** | S5 | B4 | PTTA | 102 | 480 | 1:15 |

To demonstrate that the level of PEG modification of PEG-modified HPAEs of the invention can be controlled, a series of PEG-modified HPAEs were prepared as shown in Table 2.

PEG-modified HPAEs were prepared according to the method described above, reacting an amine-endcapped HPAE with an Mw of 15-25 kDA and having an average of 25 amine terminals with a PEG methyl ether acrylate with an Mn of 1500-2000 kDa. The level of PEG modification in the resulting product was assessed by 1H NMR spectroscopy.

For entries 1-3, the level of PEG modification was controlled by altering the feeding ratio of HPAE and PEG methyl ether acrylate. For entries 4-6, the level of PEG modification was controlled by altering the reaction time. For entries 7-9, the level of PEG modification was controlled by altering the reaction temperatures. Lastly, for entries 10-12, the level of PEG modification was controlled by altering reaction temperature.

**Table 2 - Controlling the level of PEG-modification**

| **Entry** | **Time / h** | **Temp. / °C** | **Concentration of HPAE in the reactior solution / mg/mL** | **Weight ratio of HPAE PEG methyl ether acrylate** | **Number of PEG substituents per HPAE molecule*** |
|---|---|---|---|---|---|
| **1** | 18 | 25 | 10 | 2:1 | 3 |
| **2** | 18 | 25 | 10 | 1:5 | 6 |
| **3** | 18 | 25 | 10 | 1:10 | 8 |
| **4** | 18 | 25 | 10 | 2:1 | 3 |
| **5** | 36 | 25 | 10 | 2:1 | 4 |
| **6** | 48 | 25 | 10 | 2:1 | 5 |
| **7** | 18 | 25 | 10 | 2:1 | 3 |
| **8** | 18 | 25 | 30 | 2:1 | 5 |
| **9** | 18 | 25 | 50 | 2:1 | 6 |
| **10** | 18 | 25 | 10 | 2:1 | 3 |
| **11** | 18 | 50 | 10 | 2:1 | 8 |
| **12** | 18 | 80 | 10 | 2:1 | 14 |

| | | | | | |
|---|---|---|---|---|---|
| * Average number, calculated by integration of 1H NMR spectra of product. | | | | | |

A series of polyplexes were prepared with a PEG-modified HPAE as the sole polymer component. Equivalent polyplexes with non-PEG-modified HPAEs were also prepared for comparison.

To produce the single-polymer polyplexes, the relevant HPAE was directly formulated with mRNA at a polymer:nucleic acid weight ratio of 20:1. The mRNA was diluted in a 25 mM sodium acetate buffer and in a separate tube the HPAE was diluted in 25 mM sodium acetate buffer to the appropriate relative concentration. The HPAE solution was added to the mRNA solution and the resulting reaction mixture was mixed well and incubated at room temperature for 2-5 minutes.

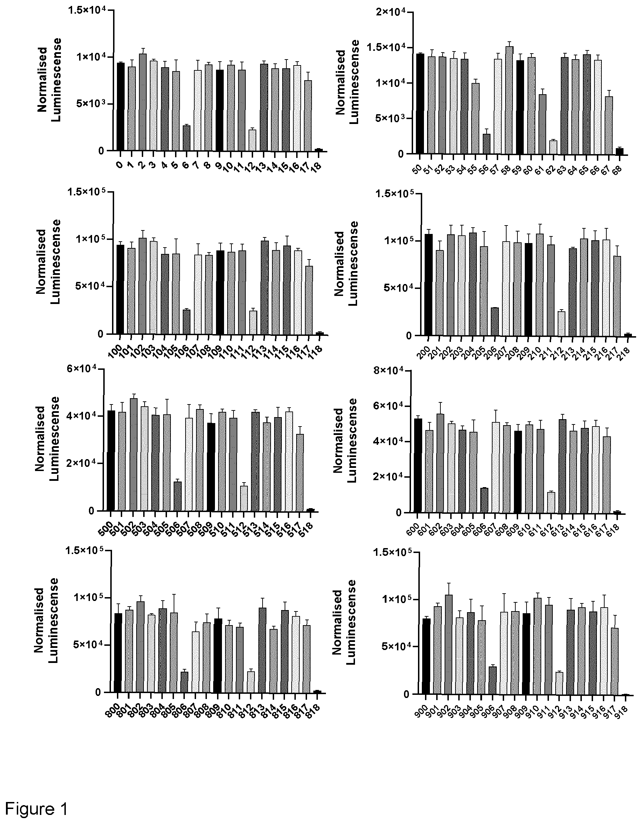

The resulting polyplexes were then freshly used for transfection of human embryonic kidney cells (HEK293). Figure 1 shows normalised luminescence intensities representing luciferase protein expression measured 48 h after transfection.

As seen from Figure 1, the level of PEG modification present did not significantly affect transfection efficiency. High transfection efficiency was achieved across almost all samples tested, with the exception of those with the highest excess of PEG modification. Furthermore, the choice of backbone and endcap monomers also played key roles in transfection efficiency.

A series of core-shell functional polyplexes, having a shell comprising PEG-modified HPAE and a core comprising the nucleic acid and a second cationic polymer, were prepared for testing. HPAE polymers without PEG-modification, whose synthesis is described above, were used as the second cationic polymer.

For all core-shell functional polyplexes tested, polyplexes were produced with an mRNA:core material:shell material (i.e. a ratio of mRNA : non-PEG-modified HPAE : PEG-modified HPAE) of 1:20:10. The mRNA was diluted in a 25 mM sodium acetate buffer and in a separate tube the non-PEG-modified HPAE was diluted in 25 mM sodium acetate buffer to the appropriate relative concentration. The non-PEG-modified HPAE solution was added to the mRNA solution and the resulting reaction mixture was mixed well and incubated at room temperature for 2-5 minutes. A solution of the PEG-modified HPAE shell material at the appropriate relative concentration was prepared in a separate tube then added to the reaction mixture and incubated at room temperature for a further 2-5 minutes. The resulting polyplexes were either freshly used for transfection, frozen, or lyophilised for long term storage.

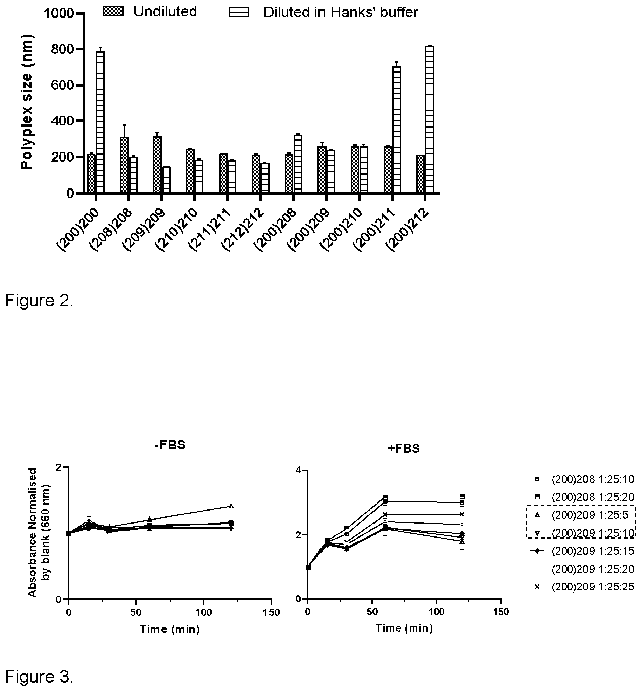

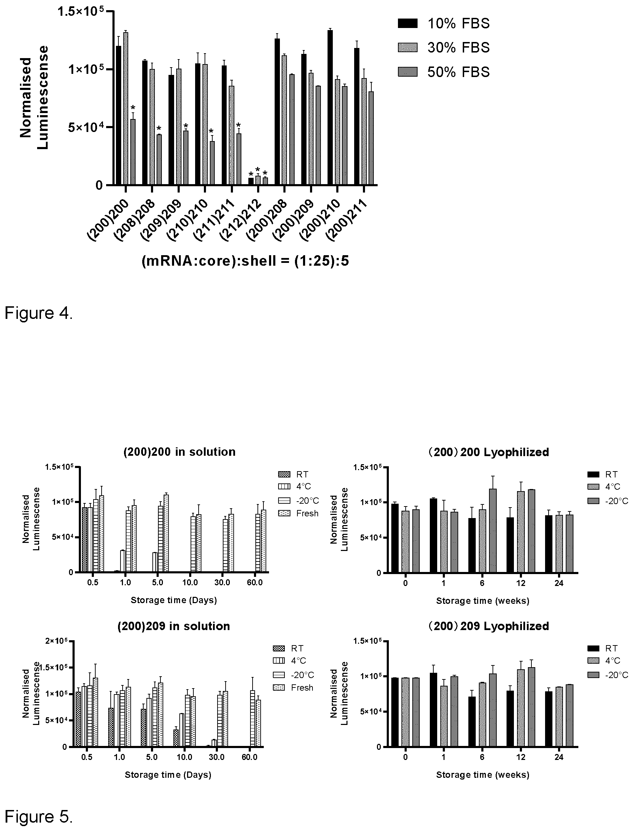

Several core-shell functional polyplex candidates were selected for testing of salt/serum stability (Figures 2 and 3) and cell transfection efficacy (Figure 4). The polymer name (No.) in brackets indicates the core material, and the polymer name (No.) outside brackets indicates the shell material. For example, in Figures 2 and 3, (200)200 refers to a polyplex made using non-PEG-modified HPAE 200 both as the second cationic polymer to form the core and as the shell material, in order to compare with e.g. (200)209, which uses 200 as the core material and PEG-modified HPAE 209 as the shell material.

The results of salt stability testing are shown in Figure 2. Polyplexes were formulated as described above and then their size distribution was measured using a Zetasizer, either freshly or after having been diluted ten times in Hanks' buffer. The addition of PEG-modified HPAEs was seen to increase polyplexes' salt stability.

To assess serum stability, a turbidity assay was used (Figure 3). Core-shell functional polyplexes (200)208 and (200)209, which had demonstrated good salt stability, were formulated as described above in a range of exemplary weight ratios of mRNA:core material:shell material. Polyplexes were first dissolved in phosphate-buffered saline (PBS), then 20% (v/v) fetal bovine serum (FBS) was added. Turbidity was measured by 660 nm absorbance to avoid FBS absorbance and all absorbance was normalized by time 0 (without the addition of FBS). The result is in accordance with the *in vivo* result.

The core-shell functional polyplexes were also used for transfection of human embryonic kidney cells (HEK293), with different serum (FBS) percentages present at transfection. Figure 4 shows normalised luminescence intensities measured 48 h after transfection.

For transfection with different serum levels, cells were seeded 24 h prior to transfections to allow attachment to well plates. Cells were seeded at optimized cell densities. On the day of transfection, polymer-mRNA complexes were prepared and, after complexation, were mixed with the appropriate cell media containing different levels of serum (Fetal Bovine Serum, FBS) such that the final polyplex solution was no more than 20% of the overall media volume. Cell media containing the polyplex were added to cells and after 4 h were removed and replaced with fresh media to remove complexes.

The addition of PEG-modified HPAEs was seen to significantly increase the serum stability of the polyplexes tested. Moreover, the transfection results showed no significant reduction in cell viability in any of the groups tested. When PEG-modified HPAEs were used as shell materials, high transfection efficiency was maintained even at the highest serum levels tested.

The thermostability of polyplexes of the invention was also investigated. Core-shell polyplex (200)209, formulated with an mRNA:core material:shell material weight ratio of 1:25:5, was used for thermostability testing. Polyplex (200)200, with no PEG modification, was used for comparison.

Samples were tested after being stored in solution or lyophilised. For lyophilisation, trehalose was added to samples of the polyplex solution to make a final trehalose concentration of 10% (w/w). All samples were frozen at -80 °C overnight and then immediately subjected to freeze-dry with a Christ Alpha 1-2 LDplus Freeze-Dryer at -55 °C for 24 h. The polyplexes were then stored at room temperature, 4 °C or -20 °C for 24 hours, or 1, 6, 12, 24 weeks, respectively. For testing of polyplexes stored in solution, trehalose was added to the polyplex solution samples to a concentration of 10% (w/w), and then directly stored at room temperature, 4 °C or -20 °C for the indicated periods.

Figure 5 shows the results of transfection of HEK293 cells, carried out as described above, for the stored polyplexes after various periods of time. Before transfection, the lyophilised samples were reconstituted with the original volume of pure water. Frozen samples were thawed. Freshly formulated polyplexes were also used as controls.

As shown in Figure 5, lyophilisation preserved the *in vitro* transfection efficacy of both the PEG-modified core-shell functional polyplex and non-PEG-modified control polyplex. Meanwhile, while non-PEG-modified polyplex (200)200 achieved high transfection efficiency when freshly made or after storage at -20 °C, transfection efficiency decreased dramatically for samples having been stored at room temperature or at 4 °C. In comparison, PEG-modified polyplex (200)209 retained good transfection efficiency after storage at room temperature or at 4 °C for up to ten days.

The *in vivo* transfection efficiency of polyplexes of the invention in mouse models was next evaluated.

Polyplexes (200)200; (200)204; (200)209; (200)213; (500)500; (500)504; (500)509 and (500)513 were selected and formulated at an mRNA:core material:shell material weight ratio of 1:25:5. They were then either used freshly or frozen/lyophilized before being reconstituted for *in vivo* transfection tests as indicated. The fresh or reconstituted polyplex solution was administrated *via* tail vein injection at a dose of 0.5 mg mRNA per kg mouse body weight. Their efficacy is compared with *in vivo* jetPEI at the same dose of mRNA.

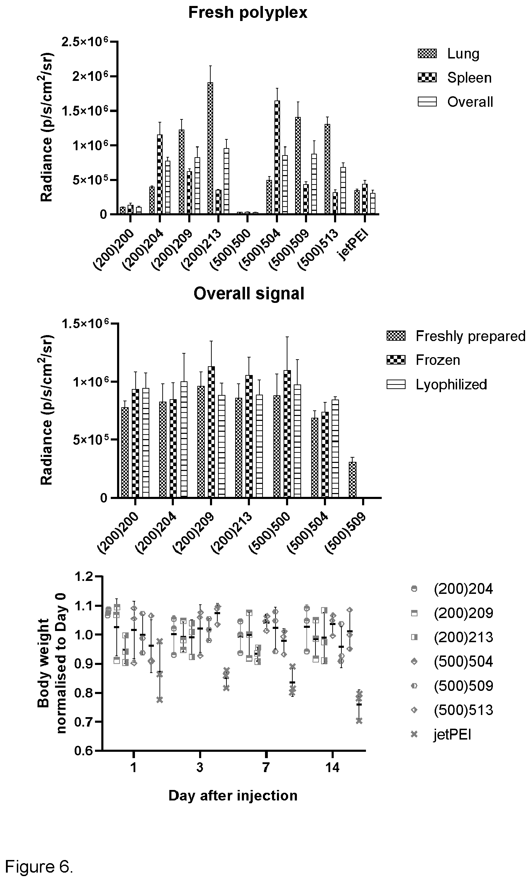

Results of the *in vivo* testing are shown in Figure 6. Groups of polyplexes formulated with luciferase-encoding mRNA were administrated intravenously in mice, and luminescence in various organs or in general was evaluated at 24 hours post-administration.

As seen from Figure 6, non-PEG-modified polyplexes demonstrated inferior efficacy compared to all PEG-modified core-shell polyplexes. It was also noted that the length of PEG may have significant impact on the targeting organs. Meanwhile, compared to *in vivo* jetPEI, no significant body weight loss was observed and significantly higher general expression was achieved after dosing, demonstrating the high potency and high biocompatibility of this type of vector for systemic delivery.

Furthermore, lyophilisation or freeze storage of PEG-modified core-shell polyplexes also preserved 100% *in vivo* transfection efficacy and caused no difference in animal body weight loss.

Figure 7 shows the effects of the level of PEG modification on polyplex size. As shown in Table 1, the PEG-acrylate:HPAE feeding ratio was altered to provide values from 1:60 to 25:2 (w/w). Examples 211 and 212, having 3:1 and 5:1 ratios respectively, showed low salt stability, potentially due to increased levels of PEG modification interrupting the core-shell interactions. Examples 1001 and 1002, having ratios of 1:60 and 1:30 respectively, also showed low salt stability.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A method for the PEG modification of HPAEs, comprising the steps of:
(a) providing a hyperbranched PAE polymer;
(b) mixing the hyperbranched polymer with a poly(ethylene glycol) monomer in a solvent;
(c) reacting the resulting mixture to provide a PEG-modified hyperbranched poly((3-amino ester) (HPAE);
wherein the level of PEG modification of the PEG-modified HPAE is controlled by one or more of the following parameters:
(i) the reaction temperature;
(ii) the reaction time;
(iii) the feeding ratio of HPAE and PEG monomer;
(iv) the reaction concentration.

2. A method according to claim 1 wherein the hyperbranched polymer is an HPAE made by reacting together:
(ii) at least one four-branching monomer having four reaction sites that can react with acrylate or amine groups;
(iii) at least one diacrylate component, typically of formula (I) 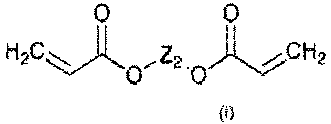 wherein Z2 is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms; wherein Z2 is unsubstituted or substituted with at least one of a halogen, a hydroxyl, an amino group, a sulfonyl group, a sulphonamide group, a thiol, a C1-C6 alkyl, a C1-C0 alkoxy, a C1-C6 ether, a C1-C6 thioether, a C1-C6 sulfone, a C1-C6 sulfoxide, a C1-C6 primary amide, a C1-C6 secondary amide, a halo C1-C5 alkyl, a carboxyl group, a cyano group, a nitro group, a nitroso group, -OC(O)NR'R', -N(R')C(O)NR'R, - N(R')C(O)O-C1-C6 alkyl, C3-C6 cycloalkyl, C3-C6 heterocyclyl, C2-C5 heteroaryl and C6-C10 aryl; wherein each R' is independently selected from the group consisting of hydrogen and C1-C6 alkyl;
(iv) at least one first amine component typically comprising 3 to 20 carbon atoms, wherein said at least one first amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(O)NR'R', -N(R')C(O)NR'R, -N(R')C(O)O-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl; and
(v) at least one second amine component typically comprising 3 to 20 carbon atoms, wherein said at least one second amine component typically is unsubstituted or substituted with at least one of a halogen, a hydroxyl, an amino group, a sulfonyl group, a sulphonamide group, a thiol, a C1-C6 alkyl, a C1-C6 alkoxy, a C1-C6 ether, a C1-C6 thioether, a C1-C6 sulfone, a C1-C6 sulfoxide, a C1-C6 primary amide, a C1-C6 secondary amide, a halo C1-C6 alkyl, a carboxyl group, a cyano group, a nitro group, a nitroso group, -OC(O)NR'R', - N(R')C(O)NR'R, - N(R')C(O)O-C1-C6 alkyl, C3-C6 cycloalkyl, C3-C6 heterocyclyl, C2-C5 heteroaryl and C6-C10 aryl; wherein each R' is independently selected, from the group consisting of hydrogen and C1-C6 alkyl.

3. A method according to claim 1 or 2 wherein the at least one four-branching monomer is PTTA:

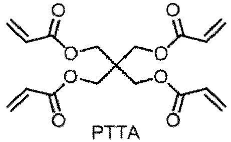

4. A method according to any preceding claim wherein the at least one diacrylate component is selected from the group comprising: 1,4-butanediol diacrylate (B4); 1,5-pentanediol diacrylate (B5):

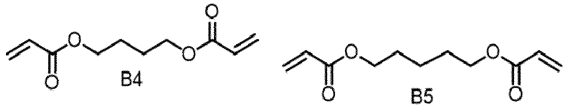

5. A method according to any preceding claim wherein the at least one first amine component is selected from the group comprising: 6-amino-1-hexanol (S6); 5-amino-1-pentanol (S5):

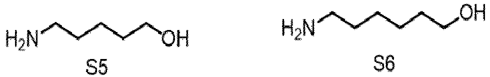

6. A method according to any preceding claim wherein the at least one second amine component is selected from the group comprising: 1,2-diaminoethane (102); 1,3-diaminopropane (103); 1,11-diamino-3,6,9-trioxaundecane (122); 1-(3-aminopropyl)-4-methylpiperazine (E7):

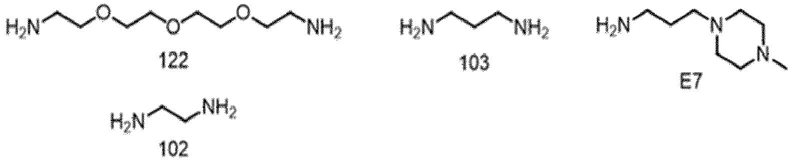

7. A method according to any preceding claim wherein the hyperbranched polymer is an HPAE which is a derivative of S5 or S6 as an amine backbone monomer, B5 or B4 as a diacrylate monomer, PTTA as a branching monomer, and 122 as an endcapping monomer.

8. A method according to any preceding claim wherein the poly(ethylene glycol) monomer is selected from the list comprising: a poly(ethylene glycol) acrylate; a poly(ethylene glycol) ether acrylate; a poly(ethylene glycol) methyl acrylate; a poly(ethylene glycol) acrylamide; a poly(ethylene glycol) epoxide; a poly(ethylene glycol) *N*-hydroxysuccinimide; a poly(ethylene glycol) carboxyl; a poly(ethylene glycol) vinyl sulfone; a poly(ethylene glycol) maleimide.

9. A method according to any preceding claim wherein the reaction is carried out at a temperature of from about 45 to about 55 °C in order to achieve a product having up to about 8 PEG substituents per repeating HPAE unit, or wherein the reaction is carried out at a temperature of from about 75 to about 85 °C in order to achieve up to about 14 PEG substituents per repeating HPAE unit.

10. A method according to any preceding claim wherein the reaction components are mixed for a reaction time of from about 30 to about 40 hours, such as 36 h, in order to achieve a product having up to about 4 PEG substituents per repeating HPAE unit, or the reaction components are mixed for a reaction time of from about 40 to about 50 hours, such as 48 h, in order to achieve a product having up to about 5 PEG substituents per repeating HPAE unit, or the reaction is carried out at an HPAE concentration of from about 25 to about 35 mg/mL, such as about 30 mg/mL, in order to achieve a product having up to about 5 PEG substituents per repeating HPAE unit.

11. A method according to any preceding claim wherein the reaction is carried out at an HPAE concentration of from about 45 to about 55 mg/mL, such as about 50 mg/mL, in order to achieve up to about 6 PEG substituents per repeating HPAE unit.

12. A method according to any preceding claim wherein the reaction components are mixed at an HPAE:PEG weight feed ratio of from about 1:4 to about 1:6, such as about 1:5, in order to achieve a product having up to about 6 PEG substituents per repeating HPAE unit, or at an HPAE:PEG weight feed ratio of from about 1:8 to about 1:12, such as about 1:10, in order to achieve a product having up to about 10 PEG substituents per repeating HPAE unit.

13. A PEG-modified hyperbranched poly(β-amino ester) (HPAE) prepared according to the method of any preceding claim.

14. A polyplex of a nucleic acid and a PEG-modified hyperbranched poly(β-amino ester) (HPAE), optionally wherein the PEG-modified hyperbranched poly(β-amino ester) (HPAE) is prepared by a method of any of claims 1-12.

15. A polyplex according to claim 14 wherein the nucleic acid and the PEG-modified HPAE are present in a weight ratio of from about 1:1 to about 1:200 respectively, suitably from about 1:5 to about 1:50 respectively, preferably from about 1:10 to about 1:40 respectively, such as about 1:20 or about 1:20 respectively.

16. A polyplex according to any of claims 14 to 15 comprising a second cationic polymer, optionally wherein the second cationic polymer is a linear poly(β-amino ester)s (LPAE) or a hyperbranched poly(β-amino ester) (HPAE), or an LPAE or HPAE without PEG modification.

17. A polyplex according to any of claims 14 to 16 having a core-shell structure, wherein the polyplex has a shell comprising PEG-modified HPAE and a core comprising the nucleic acid and a second cationic polymer, optionally wherein the core comprises the nucleic acid, LPAE or HPAE without PEG modification and a shell comprising PEG-modified HPAE.

18. A polyplex according to any of claims 16 to 17 wherein the PEG-modified HPAE and the second cationic polymer may be present in the polyplex in a weight ratio of from about 5:1 to about 1:100 respectively, suitably from about 1:1 to about 1:20 respectively, for example from about 1:2 to about 1:10 respectively, for example about 1:2 respectively or about 1:5 respectively.

19. A method of *in vivo or in vitro* transfection comprising the use of a polyplex according to any of claims 14 to 18 as a vector.

20. A method for producing a polyplex of a nucleic acid and a PEG-modified HPAE comprising the step of mixing a nucleic acid with a PEG-modified HPAE, optionally wherein the nucleic acid and the PEG-modified HPAE are mixed in a weight ratio of from about 1:5 to about 1:50 respectively, suitably from about 1:10 to about 1:40 respectively, preferably from about 1:15 to about 1:30 respectively.

21. A method according to claim 20 further comprising an earlier step of mixing the nucleic acid with a second cationic polymer to form a core, which is then mixed with the PEG-modified HPAE, optionally the core and the PEG-modified HPAE are mixed in a weight ratio of from about 0:1 to about 100:1 respectively.
